# EUROPÄISCHE PATENTSCHRIFT

(11) **EP 0 502 258 B1**
(45) Veröffentlichungstag und Bekanntmachung des Hinweises auf die Patenterteilung: **23.07.1997**
(21) Anmeldenummer: 91122160.4
(22) Anmeldetag: 23.12.1991
(51) Int. Cl.: A61F 9/00, A61B 17/02

(54) **Operationshaken zur Verwendung beim Eingriff am Auge eines Lebewesens**
Hook for use in eye surgery
Crochet à utiliser dans la chirurgie de l'oeil

(30) Priorität: 06.03.1991 CH 679/91
(43) Veröffentlichungstag der Anmeldung: 09.09.1992
(73) Patentinhaber: DUKE UNIVERSITY MEDICAL CENTER, Durham, North Carolina 27710 (US); GRIESHABER & CO. AG SCHAFFHAUSEN, CH-8203 Schaffhausen (CH)
(72) Erfinder: Cobo, Michael, M.D., Durham, NC 27701 (US); Hickingbotham, Dyson, Bahama, NC 27503 (US); De Juan, Eugene, Jr., Durham, NC 27707 (US)
(74) Vertreter: Althoff, Gerhard

(56) Entgegenhaltungen:
- EP-A- 0 156 218
- US-A- 4 037 589
- US-A- 4 616 633
- AMERICAN JOURNAL OF OPHTHALMOLOGY Bd. 110, Nr. 5, 15. November 1990, Seite 557; DWAIN G. FULLER ET AL.: 'Translimbal iris hook for pupillary dilation during vitreous surgery'

## Beschreibung

Die Erfindung bezieht sich auf einen Operationshaken zur Verwendung beim Eingriff am Auge eines Lebewesens, bestehend aus einem in Form eines länglichen Profilkörpers ausgebildeten und mindestens an dem einen Ende mit einem in die Vorderkammer des Auges einführbaren und zum Einhängen und Zurückziehen der Iris hakenförmig ausgebildeten Einhängeteil versehenen Führungskörper sowie einem flexiblen und mit einer Durchtrittsöffnung versehenen Klemmteil, welches an dem Führungskörper in Längsrichtung verschiebbar angeordnet und zur Fixierung desselben etwa formschlüssig anliegend der Cornea zuführbar ist.

Für operative Eingriffe am Auge eines Lebewesens ist aus der US-A 4,037,589 eine Rückhalteeinrichtung bekannt, welche im wesentlichen ein Drahtgestell mit zwei umgebogen angeformten und gegenüberliegend angeordneten Rückhalteelementen für das wahrend des operativen Eingriffs in einer geöffneten Stellung zu haltende Augenlid und einen an dem Drahtgestell angeordneten ersten Operationshaken sowie zwei beabstandete und funktionell unabhängige zweite Operationshaken umfasst, wobei die Operationshaken jeweils unabhängig voneinander durch verteilt in der Cornea vorgesehene Einschnitte zum Einhängen und Zurückziehen der Iris in die Vorderkammer einführbar sind. Der zweite Operationshaken hat einen aus einem hakenförmig umgebogenen Draht gebildeten Führungskörper sowie eine daran in Längsrichtung verschiebbare und aus elastisch verformbarem Material hergestellte Scheibe, welche zum Abdichten des einzelnen Einschnitts sowie zum Fixieren des Operationshakens der äusseren Kontur der Cornea zuführbar und mit verhältnismässig grosser Flächenpressung daran angeordnet ist.

Ein weiterer Operationshaken sowie dessen Anwendung ist aus der druckschriftlichen Veröffentlichung (AMERICAN JOURNAL OF OPHTHALMOLOGY, Bd. 110, Nr.5, Dwain G.Fuller M.D. et al.; Translimbal Iris Hook for Pupillary Dilation During Vitreous Surgery) bekannt. Der aus einem Draht geformte und an den Enden jeweils hakenförmig umgebogene Führungskörper des Operationshakens wird jeweils durch einen entsprechend vorgesehenen Einschnitt in die Vorderkammer eingeführt und nach dem Zurückziehen der Iris mit einem in Längsrichtung an dem Führungskörper verschiebbaren, zylindrisch ausgebildeten Klemmkörper gehalten. Auch bei dieser Variante ist die Flächenpressung des bündig am Aussenrand der Cornea anliegenden Klemmkörpers relativ gross und zudem müssen hierbei die einzelnen zum Einführen des Klemmkörpers in der Cornea vorgesehenen Einschnitte nach dem operativen Eingriff und Entfernen desselben durch relativ zeitaufwendiges Nähen wieder geschlossen werden.

Der Erfindung liegt die Aufgabe zugrunde, einen Operationshaken gemäss der im Oberbegriff des Patentanspruchs 1 genannten Gattung dahingehend auszubilden und zu verbessern, dass unter Beibehaltung einer präzisen Handhabung und Einführung in die Vorderkammer sowie beim Einhängen und Zurückziehen der Iris und in zurückgezogenem Zustand derselben eine exakte Fixierung des Operationshakens mit möglichst geringer Flächenpressung an der äusseren Kontur der Cornea gewährleistet ist.

Diese Aufgabe wird gemäss der Erfindung dadurch gelöst, dass der Führungskörper als ein mit mindestens zwei in parallelem Abstand zueinander angeordneten sowie in Längsrichtung orientierten Führungsflächen und/oder orthogonal dazu angeordneten Führungsstegen versehener stabförmiger Profilkörper und das daran verschiebbar angeordnete Klemmteil als rechteckiges Plättchen ausgebildet ist, welches mit mindestens einer quer zu dessen Längsseiten orientierten und analog dem Profilquerschnitt des Führungskörpers ausgebildeten Durchtrittsöffnung und im Bereich derselben entweder mit einer zentral angeordneten oder aber an beiden Längsseiten mit je einer das Klemmteil in vertikaler Richtung durchdringenden Ausnehmung versehen ist.

Mit der erfindungsgemässen Ausgestaltung des Klemmteils wird eine weitgehend reibungsfreie und exakte Fixierung des kompletten Operationshakens an der äusseren Kontur der Cornea erreicht, ohne dass beim operativen Eingriff ein Einreissen der Iris beziehungsweise ein Einreissen und eine unerwünschte Vergrösserung des jeweiligen Einschnitts in der Cornea entsteht.

Der als stabförmiger Profilkörper ausgebildete Führungskörper gewährleistet zudem eine in Längsrichtung desselben orientierte, exakte Schiebebewegung und formschlüssige Anlage des Klemmteils an der äusseren Kontur der Cornea sowie eine lagestabile Halterung des Operationshakens.

Im Rahmen der Erfindung hat sich weiterhin herausgestellt, dass der einzelne Einschnitt sich nach dem Herausziehen des erfindungsgemässen Operationshakens weitgehend selbsttätig verschliesst, so dass ein Austreten der Kammerflüssigkeit weitgehend verhindert und ein zeitaufwendiges Vernähen der Einschnitte gar nicht oder aber nur noch in seltenen Fällen erforderlich ist. Weiterhin wird durch die kombinierte Ausgestaltung des Klemmteils mit zwei daran angeordneten Führungsteilen erreicht, dass ein relativ grosser Bereich der Iris zurückgezogen und fixiert werden kann, wobei sich hierbei die Verwendung von Führungsteilen, bei welchen das freie und in den Raum ragende Teilstück etwa der Sklera bogenförmig ausgebildet ist, als besonders vorteilhaft herausgestellt hat.

Weitere Merkmale der Erfindung ergeben sich aus der folgenden Beschreibung in Verbindung mit den in der Zeichnung dargestellten Ausführungsbeispielen.

Die Erfindung wird nachstehend anhand der Zeichnung beschrieben. Es zeigt:
- Fig.1: ein schematisch und als Horizontalschnitt dargestelltes Auge, bei welchem der eine Bereich der Iris mit einem am Aussenrand der Cornea gehaltenen Operationshaken zurückgezogen ist;
- Fig.2: ein in Draufsicht dargestelltes Teilstück des Auges mit dem anhand des Operationshakens zurückgezogenen Bereich der Iris;
- Fig.3: ein in Draufsicht und in grösserem Massstab dargestelltes Klemmteil für den Operationshaken;
- Fig.4: das in Ansicht dargestellte Klemmteil gemäss Fig.4,
- Fig.5: einen in Ansicht und in grösserem Massstab dargestellten Führungskörper mit daran angeordnetem Einhängeteil für den Operationshaken;
- Fig.6: eine weitere Ausführungsform des am vorderen Teilstück des Führungskörpers angeordneten Einhängeteils für den Operationshaken;
- Fig.7: eine in Draufsicht dargestellte zweite Ausführungsform des Klemmteils für den Operationshaken;
- Fig.8: ein in Draufsicht dargestelltes Teilstück des Auges, bei welchem gemäss einer weiteren Variante die Iris mit einem kreisbogenförmigen Klemmteil und zwei Operationshaken zurückgezogen ist; und
- Fig.9: das in Draufsicht dargestellte Auge mit mehreren in Umfangsrichtung verteilt angeordneten Operationshaken.

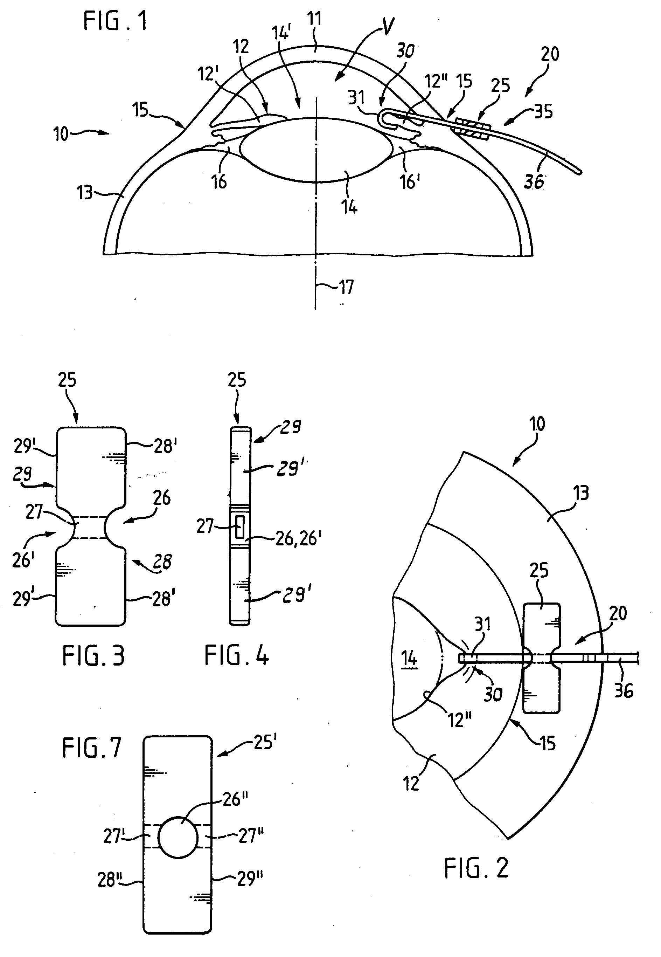

Zur Verdeutlichung der Erfindung ist in Fig.1 ein Auge 10 in schematischem Horizontalschnitt dargestellt und man erkennt die Hornhaut 11 (Cornea), die Regenbogenhaut 12 (Iris) mit den beiden Bereichen 12' und 12'', die Lederhaut 13 (Sklera), die Linse 14 (Okular), die Pupille 14' sowie die Strahlenbänder 16, 16' (Zonula).
Weiterhin erkennt man in Fig.1 einen in der Gesamtheit mit 20 bezeichneten Operationshaken, mittels welchem beispielsweise der eine Bereich 12'' der Iris 12 relativ zu der theoretischen Seh-Achse 17 des Auges 10 nach aussen gezogen und etwa an dem mit 15 bezeichneten Übergang von der Cornea 11 zur Sklera 13 gehalten ist. Der Operationshaken 20 umfasst im wesentlichen ein plättchenförmig ausgebildetes, in Fig.1 im Profilquerschnitt dargestelltes Klemmteil 25 sowie einen Führungskörper 35, welcher mit einem daran angeordneten Einhängeteil 30 die Cornea 11 durchdringt und am Bereich 12'' der Iris 12 eingehangt ist.

Fig.2 zeigt in schematisch dargestellter Draufsicht ein Teilstück des Auges 10 mit dem anhand des Operationshakens 20 zurückgezogenen Bereich 12'' der Iris 12. Der Operationshaken 20 ist dabei mit dem verschiebbar am Führungskörper 35 angeordneten und am Übergang 15 des Auges 10 anliegenden Klemmteil 25 gehalten. Das Klemmteil 25 ist aus flexiblem Material hergestellt, so dass sich die dem Übergang 15 zugewandte Längsseite entsprechend dem kreisbogenförmigen Übergang 15 der Cornea 11 anpassen kann (nicht dargestellt).

Die Ausgestaltung sowie weitere Varianten des Klemmteils 25 und des mit einem Einhängeteil 30 versehenen Führungskörpers 35 für den Operationshaken 20 werden nachstehend anhand der Figuren 3 bis 8 beschrieben.
Das in Fig.3 in Draufsicht und in Fig.4 in Seitenansicht dargestellte, plättchenförmig ausgebildete Klemmteil 25 wird von einer etwa schlitzförmig ausgebildeten, quer zu den Längsseiten 28,29 orientierten Durchtrittsöffnung 27 durchdrungen. Im Bereich der Durchtrittsöffnung 27 ist das im Profilquerschnitt vorzugsweise rechteckig ausgebildete Klemmteil 25 mit zwei gegenüberliegend zueinander an den Längsseiten 28,29 angeordneten Ausnehmungen 26,26' versehen. Die beiden den Profilquerschnitt des Klemmteils 25 in vertikaler Richtung durchdringenden Ausnehmungen 26,26' sind beispielsweise kreisbogenförmig ausgebildet, wodurch die beiden Längsseiten 28,29 jeweils in zwei Teilstücke 28' und 29' unterteilt sind. Die mit den Ausnehmungen 26,26' versehenen Längsseiten 28 und 29 gewährleisten eine optimale Anpassung des Klemmteils 25 an dem kreisbogenförmigen Übergang 15 des Auges 10.
Durch die beiden Ausnehmungen 26,26' wird zudem in diesem Bereich eine Verringerung des Profilquerschnitts und der Länge der schlitzförmigen Durchtrittsöffnung 27 erreicht, wodurch die Reibung an dem durch die Durchtrittsöffnung 27 hindurchschiebbaren Führungskörper 35 verringert und die Handhabung des Operationshakens 20 verbessert wird. Die schlitzförmige Durchtrittsöffnung 27 (Fig.4) in dem Klemmteil 25 ist entsprechend dem Profilquerschnitt des Führungskörpers 35 ausgebildet.
Das Klemmteil 25 dient im wesentlichen zum Positionieren und Festhalten des Führungskörpers 35 am Übergang 15 des Auges 10. Die dabei dem Übergang 15 bezeihungsweise der Cornea 11 zugewandte Längsseite 28 oder 29 des Klemmteils 25 kann sich aufgrund der Ausnehmung 26 oder 26' und der Flexibilität des Klemmteils 25 in nicht näher dargestellter Weise der Kontur beziehungsweise dem Übergang 15 der Cornea 11 anpassen.

Fig.7 zeigt als weiteres Ausführungsbeispiel ein in Draufsicht dargestelltes Klemmteil 25', bei welchem anstelle der beiden seitlichen Ausnehmungen 26,26' (Fig.3) eine einzige, zentral angeordnete Ausnehmung 26'' vorgesehen ist. Die Ausnehmung 26'' steht mit zwei miteinander korrespondierenden Durchtrittsöffnungen 27' und 27'' in Verbindung, durch welche der Führungskörper 35 weitgehendreibungslos hindurchgeführt werden kann.

Bei einem weiteren nicht dargestellten Ausführungsbeispiel des Klemmteils 25 oder 25' kann eine der beiden Längsseiten 28 oder 29 mit einer dem Übergang 15 beziehungsweise der Kontur der Cornea 11 entsprechend ausgebildeten Kreisbogenform versehen sein.

Das Klemmteil 25 oder 25' ist etwa als rechteckiges Plättchen ausgebildet, welches mit mindestens einer quer zu beiden Längsseiten 28 und 29 (Fig.3) oder 28'' und 29'' (Fig.7) den Profilquerschnitt des Klemmteils 25 oder 25' durchdringenden und dem Profilquerschnitt des Führungskörpers 35 entsprechend schlitzförmig ausgebildeten Ausnehmung 27 (Fig.3,4) oder 27',27'' (Fig.7) versehen ist.

Das Klemmteil 25 oder 25' ist aus geeignetem flexiblen Kunststoff hergestellt, welcher zudem optimale Gleiteigenschaften zum weitgehend kraftlosen Durchschieben des Führungskörpers 35 aufweist. Das Klemmteil 25 oder 25' ist vorzugsweise aus Silikon (Silikon-Kautschuk) hergestellt.

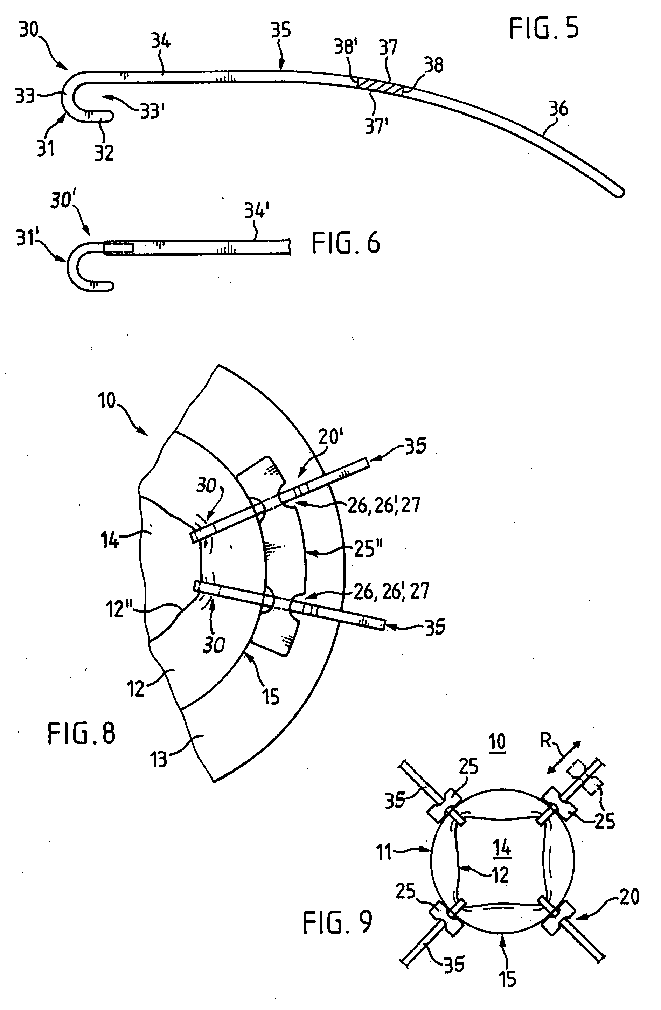

Der in Fig.5 in Ansicht dargestellte und in der Gesamtheit mit 35 bezeichnete Führungskörper umfasst das Einhängeteil 30 in Form eines einmal haarnadelförmig umgebogenen Hakenstückes 31. Bei einem bevorzugten Ausführungsbeispiel hat der Führungskörper 35 ein mit dem Hakenstück 31 versehenes und gerade ausgebildetes Teilstück 34 sowie ein etwa bogenförmig ausgebildetes Teilstück 36. An dem einen Ende des geraden Teilstücks 34 ist das mit einem Bogen 33 und einem etwa in parallelem Abstand 33' zum Teilstück 34 angeordneten Schenkel 32 versehene Hakenstück 31 des Einhängeteils 30 angeformt.
Der Führungskörper 35 mit den Teilen 30,34 und 36 ist vorzugsweise als stabförmiger Profilkörper ausgebildet und hat mindestens zwei in parallelem Abstand zueinander angeordnete und in Längsrichtung orientierte Führungsflächen 37,37' und/oder orthogonal dazu angeordnete, seitliche Führungsstege 38,38'. An den Flächen 37,37' und/oder Stegen 38,38' ist das Klemmteile 25 in Längsrichtung des Führungskörpers 35 verschiebbar geführt.
Der die Teile 30,33,34 und 36 umfassende Führungskörper 35 kann im Profilquerschnitt quadratisch oder, wie in Fig.5 im Profilquerschnitt dargestellt, rechteckig ausgebildet sein. Eine bevorzugte und im Profilquerschnitt rechteckige, relativ flache Formgebung des Führungskörpers 35 gewährleistet eine präzise Handhabung beim Einführen in die Vorderkammer V des Auges 10 (Fig.1) sowie beim Entfernen desselben und verhindert zudem ein Einreissen der sehr empfindlichen Iris.

Das in den freien Raum ragende, bogenförmige Teilstück 36 des Führungskörpers 35 (Fig1) kann vorzugsweise der Formgebung (nicht dargestellt) der äusseren Kontur der Sklera 13 angepasst werden, wodurch ein gut zugänglicher Operationsbereich gewährleistet ist.
Zur Handhabung ist der Führungskörper 35 im Endbereich des bogenförmigen Teilstücks 36 mit einer nicht dargestellten Riffelung, Kerbung oder dergleichen versehen, mittels welcher eine optimale Handhabung und Positionierung erreicht wird.
Der Führungskörper 35 mit den Teilen 30,33,34 und 36 kann aus einem nicht rostenden Stahl, beispielsweise aus geeignetem Chrom-Nickel-Federstahl (X 12 Cr.Ni 17 7; nach DIN 17224) hergestellt werden. Die Verwendung eines magnetisierten Chrom-Nickel-Stahls zur Herstellung des Führungskörpers 35 hat den Vorteil, dass dieser mit einem nicht dargestellten Instrument (Zange) besser aus dem Operationsbereich entfernt werden kann. Die Abmessungen des Führungskörpers 35 liegen in der Grössenordnung von 6-8 mm Länge und 0,4 mm Breite sowie 0,15 mm Dicke.
Der Führungskörper 35 kann jedoch auch aus einem geeigneten Kunststoff-Material hergestellt werden, bei welchem das Hakenstück 31 zum Erfassen und Halten der Iris eine ausreichende Steifigkeit und das bogenförmige Teilstück 36 zum Anpassen an die äussere Kontur der Sklera 13 relativ flexibel ausgebildet ist. Durch eine entsprechende Flexibilität des verwendeten Kunststoffs ist die Verletzungsgefahr im Inneren des Auges 10 durch unbeabsichtigtes Berühren des herausragenden Teilstücks 35,36 weitgehend ausgeschlossen. Der aus geeignetem Kunststoff hergestellte Führungskörper 35 kann zudem ohne besonderen Aufwand mit dem Teilstück 36 an die aussere Form der Sklera 13 angepasst werden, wodurch ebenfalls ein unbeabsichtiges Berühren verhindert wird.

Bei einer in Fig.6 teilweise dargestellten Ausführungsvariante besteht die Möglichkeit, den aus geeignetem Kunststoff hergestellten Führungskörper 35 mit einem aus einem nicht rostenden Stahl, beispielsweise aus geeignetem Chrom-Nickel-Federstahl (X 12 Cr.Ni 17 7; nach DIN 17224) hergestellten Einhängeteil 30' zu versehen. Das Hakenstuck 31' des Einhängeteils 30' ist hierbei in geeigneter Weise im vorderen Teilstück 34' des im Profilquerschnitt stabförmig ausgebildeten Führungskörpers 35 eingebettet angeordnet.

Fig.8 zeigt in schematisch dargestellter Draufsicht ein Teilstück des Auges 10 mit dem anhand eines in der Gesamtheit mit 20' bezeichneten Operationshakens zurückgezogenen Iris-Bereich 12''. Bei diesem Ausführungsbeispiel sind an einem Klemmteil 25'' zwei im Abstand zueinander angeordnete Führungskörper 35 vorgesehen. Das Klemmteil 25'' entspricht im wesentlichen dem Klemmteil 25 gemäss Fig.3 und ist abweichend davon mit entsprechend im Abstand zueinander angeordneten Ausnehmungen 26,26' und Durchtrittsöffnungen 27 versehen. Abweichend von der Ausgestaltung gemäss Fig.3, ist das Klemmteil 25'' gemäss Fig.8 als kreisbogenförmiges Segmentteil ausgebildet, an welchem zwei im Abstand zueinander angeordnete Führungskörper 35 angeordnet sind.

Fig.9 zeigt als weitere Variante eine schematisch dargestellte Draufsicht des Auges 10 und man erkennt mehrere, zum Öffnen der Pupille 14' (Fig.1) am Umfang verteilt angeordnete Operationshaken 20. Mittels dem am Führungskörper 35 angeformten Einhängeteil 30 wird der Iris-Bereich erfasst sowie gehalten und etwa radial nach aussen gezogen. Mit dem jeweils an dem Führungskörper 35 angeordneten und in Pfeilrichtung R verschiebbaren Klemmteil 25 wird der den jeweiligen Iris-Bereich am Übergang 15 fixierende Operationshaken 20 gehalten.

## Patentansprüche

1. Operationshaken zur Verwendung beim Eingriff am Auge eines Lebewesens, bestehend aus einem in Form eines länglichen Profilkörpers ausgebildeten und mindestens an dem einen Ende mit einem in die Vorderkammer (V) des Auges (10) einführbaren und zum Einhängen und Zurückziehen der Iris (12) hakenförmig ausgebildeten Einhängeteil (30) versehenen Führungskörper (35) sowie einem flexiblen und mit einer Durchtrittsöffnung versehenen Klemmteil, welches an dem Führungskörper (35) in Längsrichtung verschiebbar angeordnet und zur Fixierung desselben etwa formschlüssig anliegend der Cornea zuführbar ist, **dadurch gekennzeichnet**, dass der Führungskörper (35) als ein mit mindestens zwei in parallelem Abstand zueinander angeordneten sowie in Längsrichtung orientierten Führungsflächen (37,37') und/oder orthogonal dazu angeordneten Führungsstegen (38,38') versehener stabförmiger Profilkörper und das daran verschiebbar angeordnete Klemmteil (25; 25';25'') als rechteckiges Plättchen ausgebildet ist, welches mit mindestens einer quer zu dessen Längsseiten (28,29) orientierten und analog dem Profilquerschnitt des Führungskörpers (35) ausgebildeten Durchtrittsöffnung (27;27',27'') und im Bereich derselben entweder mit einer zentral angeordneten oder aber an beiden Längsseiten (28,29) mit je einer das Klemmteil (25;25';25'') in vertikaler Richtung durchdringenden Ausnehmung (26,26';26'') versehen ist.

2. Operationshaken nach Anspruch 1, **dadurch gekennzeichnet**, dass die an den beiden Längsseiten (28,29) des Klemmteils (25;25'') gegenüberliegend zueinander angeordneten Ausnehmungen (26,26') jeweils ausgehend von der einen Längsseite in Richtung der gegenüberliegenden Längsseite halbkreisförmig ausgebildet sind.

3. Operationshaken nach Anspruch 1, **dadurch gekennzeichnet**, dass das Klemmteil (25;25';25'') mindestens eine etwa kreisbogenförmig ausgebildete Längsseite (28,29) aufweist, welche weitgehend der zirkulären Formgebung der Cornea (11) ausgebildet ist.

4. Operationshaken nach Anspruch 1, **dadurch gekennzeichnet**, dass das Klemmteil (25'') als kreisbogenförmiges Segmentteil ausgebildet und mit zwei an den bogenförmigen Längsseiten im Abstand zueinander angeordneten sowie jeweils zur Aufnahme eines Führungskörpers (35) ausgebildeten Durchtrittsöffnungen (27) versehen ist.

5. Operationshaken nach Anspruch 4, **dadurch gekennzeichnet**, dass den beiden im Abstand zueinander angeordneten Durchtrittsöffnungen (27) jeweils zwei gegenüberliegend zueinander angeordnete und das Klemmteil (25'') in vertikaler Richtung durchdringende Ausnehmungen (26,26') zugeordnet sind, welche ausgehend von der einen bogenförmigen Längsseite in Richtung der gegenüberliegenden bogenförmigen Längsseite halbkreisförmig ausgebildet sind.

6. Operationshaken nach einem der Ansprüche 1 bis 5, **dadurch gekennzeichnet**, dass das als rechteckiges Plättchen ausgebildete und mit den Ausnehmungen (26,26';26'') sowie mit mindestens einer Durchtrittsöffnung (27) versehene Klemmteil (25; 25';25'') aus flexiblem Kunststoff, beispielsweise aus Silikon mit guten Gleiteigenschaften hergestellt ist.

7. Operationshaken nach Anspruch 1, **dadurch gekennzeichnet**, dass der an dem einen Ende mit dem einmal haarnadelförmig umgebogenen Einhängeteil (30;30') versehene Führungskörper (35) an dem anderen Ende mit einem ersten und zweiten Teilstück (34,36) zur Führung des Klemmteils (25;25';25'') versehen ist, wobei das zweite Teilstück (36) relativ zu dem ersten Teilstücke (34) etwa analog der Kontur der Sklera (13) bogenförmig ausgebildet ist.

8. Operationshaken nach Anspruch 7, **dadurch gekennzeichnet**, dass der das Einhängeteil (30) sowie die daran angeformten Teilstücke (34,36) umfassende Führungskörper (35) aus Chrom-Nickel-Federstahl oder aber aus Kunststoff hergestellt und im Profilquerschnitt rechteckig oder quadratisch ausgebildet ist.

9. Operationshaken nach Anspruch 8, **dadurch gekennzeichnet**, dass der aus Kunststoff hergestellte Führungskörper (35) mit einem aus Chrom-Nickel-Federstahl hergestellten Einhängeteil (30') versehen ist, welches eingebettet am vorderen Teilstück (34) angeordnet ist.

## Claims

1. Surgical hook for use during an operation on the eye of a living being consisting of a guide member (35) designed in the form of an elongate profile member and provided, at least at one end, with a hitching part (30) which can be introduced into the anterior chamber (V) of the eye (10) and is designed in the form of a hook for hitching and retracting the iris (12) and of a flexible clamp which is provided with a through-orifice, is longitudinally displaceably arranged on the guide member (35) and, for fixing it with substantially positive contact, can be supplied to the cornea, characterised in that the guide member (35) is designed as a rod-shaped profile member with at least two longitudinally orientated guide faces (37, 37') arranged with parallel mutual spacing and/or guide webs (38, 38') arranged orthogonally thereto and the clamp (25; 25'; 25'') arranged displaceably thereon is designed as a small rectangular plate provided with at least one through-orifice (27; 27', 27'') orientated transversely to its longitudinal sides (28, 29) and designed similarly to the profile cross section of the guide member (35) and is provided in the region thereof either with a centrally arranged recess (26, 26'; 26'') or on both longitudinal sides (28, 29) with a respective recess (26, 26'; 26'') vertically penetrating the clamp (25; 25'; 25'').

2. Surgical hook according to claim 1, characterised in that the recesses (26, 26') arranged opposite one another on the two longitudinal sides (28, 29) of the clamp (25; 25'') are each semicircular in design starting from one longitudinal side in the direction of the opposing longitudinal side.

3. Surgical hook according to claim 1, characterised in that the clamp (25; 25'; 25'') has at least one longitudinal side (28, 29) which is designed substantially in the form of an arc and is designed approximately to the circular shaping of the cornea (11).

4. Surgical hook according to claim 1, characterised in that the clamp (25'') is designed as an arcuate segment part and is provided with two through-orifices (27) arranged with mutual spacing on the arcuate longitudinal sides and each designed to receive a guide member (35).

5. Surgical hook according to claim 4, characterised in that two respective mutually opposed recesses (26, 26') which vertically penetrate the clamp (25'') and are semicircular in design starting from one arcuate longitudinal side in the direction of the opposing arcuate longitudinal side are allocated to the two mutually spaced through-orifices (27).

6. Surgical hook according to one of claims 1 to 5, characterised in that the clamp (25; 25'; 25'') designed as a small rectangular plate and provided with the recesses (26, 26'; 26'') and with at least one through-orifice (27) is produced from flexible plastics material, for example from silicone having good sliding properties.

7. Surgical hook according to claim 1, characterised in that the guide member (35) provided at one end with the hitching part (30; 30') bent once in the form of a hairpin is provided at the other end with a first and second portion (34, 36) for guiding the clamp (25; 25'; 25''), wherein the second portion (36) is arcuate in design relative to the first portion (34) substantially similarly to the contour of the sclera (13).

8. Surgical hook according to claim 7, characterised in that the guide member (35) comprising the hitching part (30) and the portions (34, 36) shaped thereon is produced from chromium nickel spring steel or from plastics material and is rectangular or square in design in the profile cross section.

9. Surgical hook according to claim 8, characterised in that the guide member (35) produced from plastics material is provided with a hitching part (30') which is produced from chromium nickel spring steel and is embedded on the front portion (34).

## Revendications

1. Crochet chirurgical à utiliser pour l'intervention à l'oeil d'un être vivant constitué d'un corps de guidage (35) en forme de profilé allongé muni, au moins à l'une des extrémités, d'une partie d'accrochage (30) conçue en forme de crochet pouvant être introduit dans la chambre oculaire avant (V) de l'oeil (10) pour accrocher et rétracter l'iris, ainsi que d'un élément de blocage flexible pourvu d'une ouverture de passage et disposé à glissement longitudinal sur le support de guidage (35) et qui, en vue de la fixation de celui-ci, peut être appliqué intimement sur la cornée, **caractérisé en ce que** le corps de guidage (35) est conçu comme corps profilé en forme de barre avec deux surfaces de guidage espacées et parallèles orientées longitudinalement et/ou des entretoises (38, 38') qui y sont orthogonales, en ce que l'élément de blocage (25, 25', 25'') qui y est disposé de manière coulissante est conçu sous forme de plaquette rectangulaire comprenant au moins une ouverture de passage (27, 27', 27'') orientée transversalement par rapport à ses côtés longitudinaux (28, 29) et conçu de manière analogue à la section du profilé du corps de guidage (35) et pourvu, dans la région de celle-ci, d'une découpe (26, 26', 26'') traversant l'élément de blocage (25, 25', 25'') verticalement ou bien en son centre ou bien sur chacun des côtés longitudinaux (28, 29).

2. Crochet chirurgical selon la revendication 1, **caractérisé en ce que** les découpes (26, 26') prévues en face l'une de l'autre sur les côtés longitudinaux (28, 29) de l'élément de blocage (25, 25'') sont, partant d'un côté longitudinal en direction du côté longitudinal opposé, conçus en forme de demi-cercle.

3. Crochet chirurgical selon la revendication 1, **caractérisé en ce que** l'élément de blocage (25, 25', 25'') comporte au moins un côté longitudinal (28, 29) en arc de cercle qui est adapté sensiblement à la forme circulaire de la cornée (11).

4. Crochet chirurgical selon la revendication 1, **caractérisé en ce que** l'élément de blocage (25'') est réalisé sous forme d'une partie de segment en arc de cercle et comporte, sur les côtés longitudinaux courbes, deux ouvertures de passage (27) à distance l'une de l'autre pour recevoir, chacune, un corps de guidage (35).

5. Crochet chirurgical selon la revendication 4, **caractérisé en ce que** les deux ouvertures de passage (27) prévues à distance l'une de l'autre sont associées respectivement à deux découpes (25, 26') correspondantes prévues face à face et traversant l'élément de blocage (25'') verticalement et qui sont, partant d'un côté longitudinal courbe en direction du côté longitudinal courbe opposé conçus en forme de demi-cercle.

6. Crochet chirurgical selon l'une quelconque des revendications 1 à 5, **caractérisé en ce que** l'élément de blocage (25, 25', 25'') conçu sous forme de plaquette rectangulaire est pourvu de découpes (26, 26', 26'') et d'au moins une ouverture de passage (27) est réalisée en matière synthétique flexible par exemple en silicone avec de bonnes propriétés de glisse.

7. Crochet chirurgical selon la revendication 1, **caractérisé en ce que** le corps de guidage (35) muni, à l'une des extrémités, d'une partie d'accrochage (30, 30') retournée en épingle à cheveux est pourvu, à l'autre extrémité, d'une première et d'une seconde section (34, 36) pour le guidage de l'élément de blocage (25, 25', 25'') et en ce que la seconde section (36) est arquée par rapport à la première section (34) de manière analogue au contour de la sclérotique (13).

8. Crochet chirurgical selon la revendication 1, **caractérisé en ce que** le corps de guidage (35) comprenant la partie d'accrochage (30) et les sections (34, 36) qui en font partie, est réalisé en acier chrome-nickel ou en matière synthétique et est, en section, de profil rectangulaire ou carré.

9. Crochet chirurgical selon la revendication 8, **caractérisé en ce que** le corps de guidage (35) réalisé en matière synthétique comporte une partie d'accrochage (30') en acier chrome-nickel qui est logée dans la section avant (34).
